# EUROPÄISCHE PATENTANMELDUNG

(11) **EP 1 441 249 A1**
(43) Veröffentlichungstag der Anmeldung: **28.07.2004**
(21) Anmeldenummer: 04100182.7
(22) Anmeldetag: 21.01.2004
(51) Int. Cl.: G02B 21/00, A61B 3/12

(54) **Operationsmikroskop**

(30) Priorität: 21.01.2003 DE 10302401
(71) Anmelder: Leica Microsystems (Schweiz) AG, 9435 Heerbrugg (CH)
(72) Erfinder: Sander, Ulrich Dr., 9445, Rebstein (CH)
(74) Vertreter: Reichert, Werner F., Dr.

(57) **Zusammenfassung**

Die Erfindung betrifft ein kombiniertes Operationsmikroskop-System mit einem Operationsmikroskop (3) und einem im Aufbau modifizierten Netzhaut-Diagnosegerät mit transskleraler, gepulster Beleuchtung.

## Beschreibung

Die Erfindung betrifft ein Operationsmikroskop mit einem Strahlengang für eine Kamera.

Mit bekannten Operationsmikroskopen, zum Beispiel mit dem M841 der Firma Leica, siehe Prospekt "The Leica M841 -The Ultimate Surgical Microscope System for Ophthalmology", Publication no. 10 M1 841 Oen, Drucklegung VII. 2000, wird bei ophthalmologischen Erkrankungen im Glaskörper, auf und unter der Netzhaut (Fundus) operiert. Der Bereich erstreckt sich von der Makula bis zur Ora Serrata.

Es gibt speziell für diesen Bereich auch Netzhaut-Diagnosegeräte, wie z.B. das Netzhaut-Diagnosegerät der Firma Medibell Medical Vision Technologies Ltd./Haifa/lsrael mit der Bezeichnung "Panoret 1000- Wide-Angle Digital Retinal Camera" gemäß Prospekt ohne Drucklegungsangabe. Für Netzhaut-Diagnosegeräte allgemein sind auch die Bezeichnungen Retina-Kameras oder Funduskameras gebräuchlich und werden von verschiedenen Herstellern hergestellt.

Funduskameras werden zur Diagnose entsprechender Erkrankungen verwendet, welche hinlänglich bekannt sind.

Das Grundprinzip solcher Funduskameras ist: Der Beobachtungsstrahlengang wird mit dem Beleuchtungsstrahlengang gemeinsam durch die Patientenpupille geführt.

Die gemeinsame Strahlführung des Beobachtungs- und Beleuchtungsstrahlenganges durch die Patientenpupille ist jedoch nur sehr schwierig zu realisieren. Es werden deshalb Kompromisse in Kauf genommen.

Die Firma Medibell hat die oben angegebene digitale Retina-Kamera entwickelt, wobei der Beobachtungsstrahl von dem Beleuchtungsstrahl getrennt wurde, so dass diese Schwierigkeiten damit umgangen wurden. Die Beob-achtung erfolgt mit einer speziellen Optik in direktem Kontakt zum Patientenauge. Die Beleuchtung wird mit Hilfe einer Faserbeleuchtung transskleral (durch Sclera = Lederhaut des Auges hindurch) ins Patientenauge geleitet.

Die Lichtquelle besteht aus drei Farbdioden, die gepulst werden. Mit den drei Farblichtpulsen wird gleichzeitig die hochauflösende digitale SW-Kamera (Schwarz-Weiß-Kamera) getriggert. Durch eine Bildaufbereitung des SW-Bildes erhält man auf dem Farbmonitor ein hervorragendes Farbbild hoher Auflösung oder wahlweise- aufgrund entsprechender Echt-Zeit-Prozessor-leistung - ein Lifebild. (Eine ähnliche Anordnung, allerdings nicht mit gepulsten Farbdioden, sondern mit einer Weißlichtquelle mit Farbfilterselektion, ist aus dem U.S.-Patent 6,309,070 bekannt.)

Der Wunsch der Chirurgen ist es, ein Netzhaut-Diagnosegerät auch während einer Operation einzusetzen, also nicht nur zur separaten Diagnose. Dabei erweist sich der Einsatz des Netzhaut-Diagnosegeräts nämlich als äußerst umständlich, da die Operation mit Hilfe des Operationsmikroskops abgebrochen werden muss, um das Netzhaut-Diagnosegerät über das Patientenauge zu bringen, um die gewünschte (Zwischen-) Diagnose zu stellen. Dann wiederum muss eventuell das Netzhaut-Diagnosegerät mit dem Operationsmikroskop wieder gewechselt werden. Des Weiteren ist es der Wunsch der Ophthalmologen, nicht ein separates, monoskopisches Bild der Retina aus dem Diagnosegerät geliefert zu bekommen, sondern ein vergrößerbares, gegebenenfalls stereoskopisches.

Aus diesen Wünschen bzw. aus den gegenwärtig vorliegenden Nachteilen ergibt sich die Aufgabe, die der Erfindung zugrunde liegt: Es soll ein verbessertes Operationsmikroskop geschaffen werden, das während seiner Anwendung eine Diagnosestellung wie bei einem Netzhaut-Diagnosegerät gestattet, ohne dass das Operationsmikroskop von der Operationsposition entfernt werden muss.

Diese Aufgabe wird durch die Anwendung der Merkmale des Anspruches 1 gelöst.

Weitere Ausbildungen der Erfindung sind in den Figuren und in den abhängigen Patentansprüchen angegeben.

Anhand von Figuren wird die Erfindung symbolisch und beispielhaft näher erläutert.

Die Figuren werden zusammenhängend und übergreifend beschrieben. Gleiche Bezugszeichen bedeuten gleiche Bauteile, Bezugszeichen mit unterschiedlichen Indices geben funktionsgleiche oder ähnliche Bauteile an.

Es zeigen dabei
Fig.1 - den systematischen Aufbau eines Operationsmikroskop-Systems, in dem erfindungsgemäß ein Operationsmikroskop mit einem Netzhaut-Diagnosegerät kombiniert ist,
Fig.2 - eine Ausgestaltungsvariante der Erfindung, bei der eine gemeinsame Lichtquelle angeordnet ist und aufgrund eines Verschiebens der Lichtquelle und/oder der Lichtleiter zwischen der Speisung des jeweiligen Lichtleiters gewechselt werden kann und
Fig.3 - eine weitere Ausgestaltungsvariante der Erfindung, bei der eine gemeinsame Lichtquelle und eine optische Lichtweiche angeordnet sind.

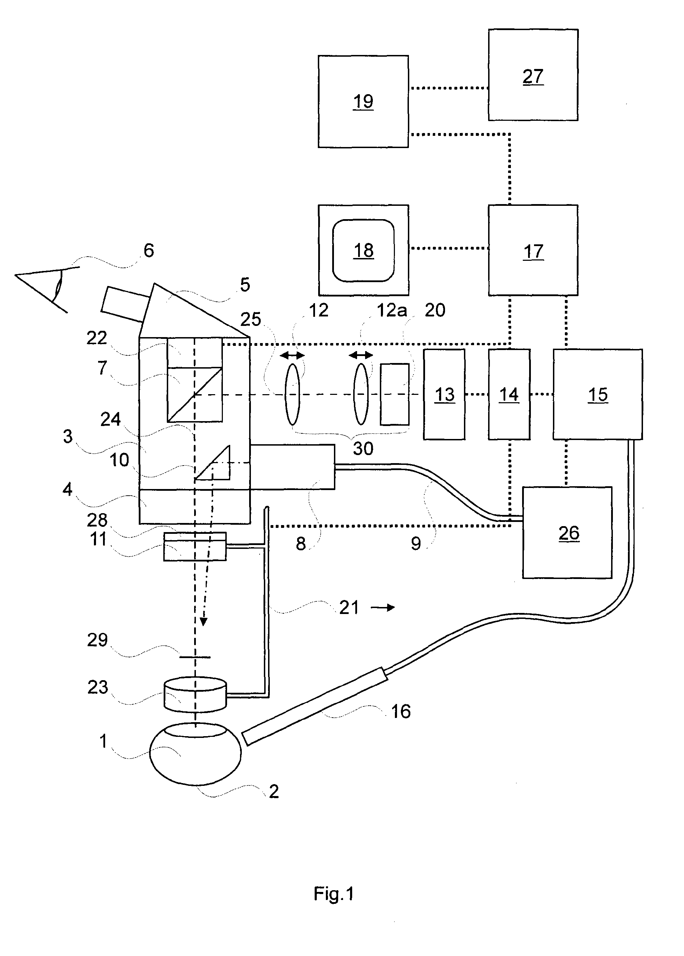

Wie aus Fig.1 ersichtlich, umfasst die erfinderische Idee, dass das Netzhaut-Diagnosegerät aus einem ersten Bauteil 13 und einem zweiten Bauteil 23 besteht. Zwischen dem ersten Bauteil 13 und dem zweiten Bauteil 23 ist ein Operationsmikroskop 3 eingebaut. Weiterhin ist aus einem Beobachtungsstrahlengang 24 mittels einem Strahlenteiler 7 ein Kamera-Strahlengang 25 ausgekoppelt, wie― beispielsweise und an sich bekannt― für Foto- oder Video-Dokumentationen üblich. Nach dem Strahlenteiler 7 ist der Kamera-Strahlengang 25 durch abbildende Linsen 12, 12a (statt einzelnen Linsen sind auch Linsengruppen einsetzbar) und durch ein Umlenkelement 20 auf eine digitale Kamera 13 gelenkt. Das dargestellte Umlenkelement 20 stellt eine mögliche optische Umlenkung dar und kann aus Spiegeln und/oder Prismen bestehen und erzeugt ein seiten- und höhenrichtiges Bild auf dem Bildsensor der Kamera 13.

Die abbildenden Linsen oder -gruppen 12, 12a können wahlweise gegeneinander verschoben werden, so dass der Abbildungsmaßstab variabel und eine separate Fokussierung möglich ist. Die beiden Linsen oder -gruppen 12, 12a bilden zusammen mit dem Umlenkelement 20 ein Abbildungssystem 30, welches wahlweise auch aus mehr als zwei Linsen oder -gruppen 12, 12a bestehen kann, die auch wahlweise fix oder axial beweglich im Kamera-Strahlengang 25 angeordnet sein können.

Die elektronischen Signale der Kamera werden über eine Bildaufbereitung 14 einem Rechner 17 zugeführt und können als Bild auf einem Monitor 18 betrachtet werden, in der Datenausgabe 19 als Druckbild ausgegeben oder auf bekannten Speichermedien gespeichert werden und können ebenso in eine zentrale Dokumentenverwaltung 27 weitergeleitet werden.

Eine stroboskopische Lichtquelle 15, mit mindestens zwei, insbesondere mit drei Farb-LEDs, wird über den Rechner 17 und die Kamera 13 so getriggert, dass nur dann Licht über einen Lichtleiter 16 auf ein Patientenauge 1 gestrahlt wird, wenn gerade eine Aufnahme mit der Kamera 13 erfolgt.

Das Operationsmikroskop 3 bildet zusammen mit dem Netzhaut-Diagnosegerät ein System, welches zwei Anwendungsmodi bietet: Einen Anwendungsmodus als Operationsmikroskop und einen Anwendungsmodus als Netzhaut-Diagnosegerät (Fundus-Modus).

Wenn das System im Fundus-Modus ist, ist vor einem Hauptobjektiv 4 eine Funduslinse 23 mit einem Strahlenvertauscher 11 und mit einer Hilfslinse 28 eingeschwenkt, die mit dem Operationsmikroskop 3 mittels einer mechanischen Einschwenkvorrichtung 21 verbunden sind. Der Strahlenvertauscher 11 vertauscht den linken und rechten Strahlengang des (Stereo-) Operationsmikroskops 3, um einen Pseudostereo-Effekt zu vermeiden, der durch die vorgeschaltete Funduslinse 23 verursacht wird. Der Strahlenvertauscher 11 kann auch wahlweise an einer anderen Stelle 22 im (Stereo-) Operationsmikroskop 3 eingebaut sein. Er wird durch den Rechner 17 aktiviert, wenn dieser in den Fundus-Modus geschaltet ist. Die Hilfslinse 28 bildet das durch die Funduslinse 23 erzeugte Zwischenbild 29 des Fundus 2 mit dem Hauptobjektiv 4 nach Unendlich ab.

Befindet sich das System nicht im Fundus-Modus, also im Anwendungsmodus des (Stereo-) Operationsmikroskops, sind die Funduslinse 23, der Strahlenvertauscher 11 und die Hilfslinse 28 nicht vor das Hauptobjektiv 4 geschwenkt. Das Patientenauge 1 wird direkt durch eine Mikroskopbeleuchtung 8, die aus einer Mikroskoplichtquelle 26 und einem Lichtleitkabel 9 besteht, über ein Umlenkelement 10 im (Stereo-) Operationsmikroskop 3 beleuchtet.

Die Mikroskoplichtquelle 26 kann vorteilhafterweise mit der Kamera-Lichtquelle 15 identisch sein, so dass das Patientenauge auch mit dem netzhautschonenden 3-Farben-LED-Stroboskoplicht beleuchtet wird. Für diesen Fall wird eine Überschreitung der Flimmergrenze des Beobachterauges 6 bevorzugt, so dass ein heller Bildeindruck entsteht, ohne jedoch zuviel Lichtenergie in das Patientenauge 1 leiten zu müssen.

Ein weiterer erfinderischer Gedanke besteht darin, dass die optische Adaption der Funduskamera 13 an das Operationsmikroskop 3 mit Hilfe eines speziellen Abbildungssystems 30 erfolgt. Wie erwähnt, besteht es aus den Linsen oder - gruppen 12, 12a und dem Umlenkelement 20. Der Erfinder erkannte, dass es ausreicht, wenn das Abbildungssystem 30 speziell nur für die Wellenlängen der mindestens zwei Farb-LEDs berechnet und korrigiert ist. Eine Farbkorrektur, wie sie für die visuelle Beobachtung im Operationsmikroskop 3 z.B. durchgeführt wird, ist in diesem Fall nicht erforderlich. Sie beschränkt sich ausschließlich auf die durch die Lichtquelle 15 angebotenen Wellenlängen und deren diskrete spektrale Verteilung. Vorteilhaft lassen sich hier erfindungsgemäß zur Reduzierung der Baulänge des Abbildungssystems 30 und des Gewichts diffraktive Elemente für die chromatische Korrektur einsetzen.

Um die Ausleuchtung der Pixel des Bildsensors der Kamera 13 optimal zu gewährleisten, ist der Strahlverlauf hinter dem Abbildungssystem 30 in Lichtrichtung so berechnet, dass eine Abschattung der Pixel durch Nachbarpixel nicht erfolgt, d.h. dass die Strahlbüschel nahezu senkrecht auf die Oberfläche der Pixel des Bildsensors auftreffen. Die mit der Korrektur des Linsensystems erreichte Auflösung muss vorteilhafterweise nicht höher sein, als es die Pixelstruktur der Kamera vorgibt.

Da im Gegensatz zu dem Abbildungssystem 30 die Funduslinse 23 und die Hilfslinse 28 auch visuell durch den Chirurgen genutzt werden, ist ihre Optik in der herkömmlichen klassischen Weise korrigiert. Die Funduslinse 23 kann aus einem System bestehen, dass in direktem Kontakt mit dem Patientenauge steht, wie es bei der Retina-Kamera der Firma Medibell der Fall ist (U.S.-Patent 6,267,752) oder es können auch die bekannten Nichtkontakt-Optiken, z.B. der Firma Okulus, verwendet werden.

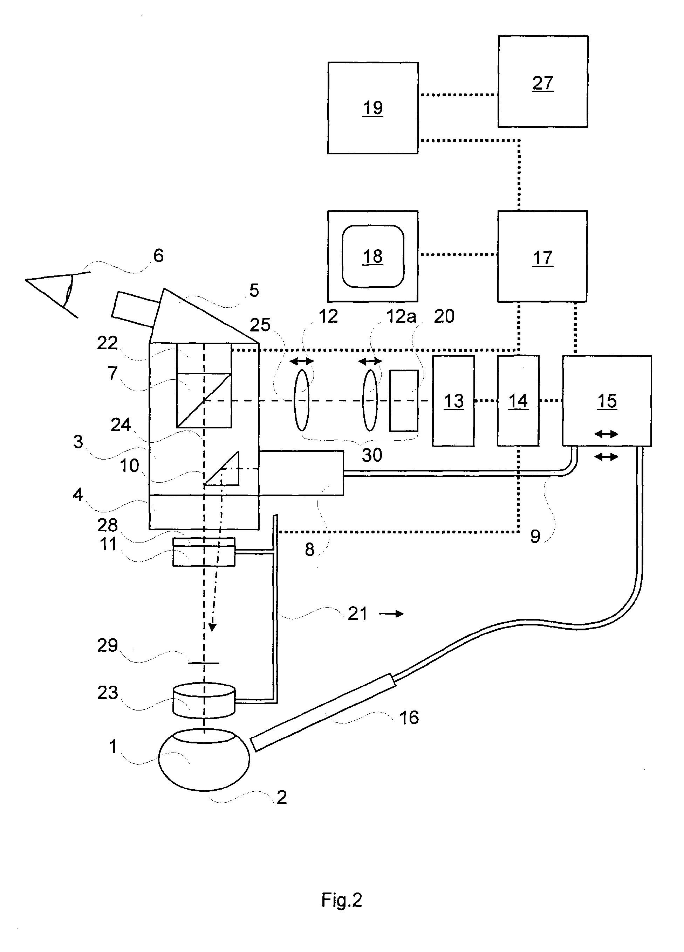

Fig.2 zeigt eine Ausgestaltungsvariante eines erfindungsgemäßen Operationsmikroskop-Systems mit einem Operationsmikroskop 3 und einem integrierten Netzhaut-Diagnosegerät , bei welchem eine einzige Lichtquelle 15 (z.B. ein 2- oder 3-Farben-LED-Stroboskoplicht) zur Speisung sowohl des Lichtleiters 9 für die herkömmliche Beleuchtung für das Operationsmikroskop 3, als auch zur Speisung des Lichtleiters 16 für die transsklerale Beleuchtung für das Netzhaut-Diagnosegerät angeordnet ist. Eine gleichzeitige Beleuchtung durch beide Lichtleiter 9 und 16 ist für den Anwendungsmodus des Netzhaut-Diagnosegeräts (Fundus-Modus) nicht vorgesehen und auch schlecht realisierbar, weil die aus dem Lichtleiter 9 gelieferte Beleuchtung für das Operationsmikroskop 3 die unter das Hauptobjektiv 4 eingeschwenkten Elemente Hilfsli n-se 28, Strahlenvertauscher 11 und die Funduslinse 23 durchscheinen müsste. Wegen dieser erfindungsgemäß angeordneten optischen Elemente für den Betrieb der Funduskamera, ist eine simultane Speisung der Lichtleiter 9 und 16 im Fundus-Modus nicht bevorzugt. Demzufolge sollen, wie schematisch dargestellt, die Lichtleiter 9 und 16 vor die Lichtquelle 15 und/oder die Lichtquelle 15 vor den je nach gewähltem Anwendungsmodus (Fundus-Modus oder Operationsmikroskop-Modus) erforderlichen Lichtleiter 9 und/oder 16 schiebbar sein. Das würde für die beiden Anwendungsmodi folgende erfindungsgemäßen Anordnungen bedeuten:

Im Fundus-Modus wird nur der Lichtleiter 16 gespeist, die Mikroskopbeleuchtung 8 liefert keine Beleuchtung, welche die eingeschwenkten Elemente Hilfslinse 28, Strahlenvertauscher 11 und Funduslinse 23 durchdringen müsste.

Im Operationsmikroskop-Modus sind die optischen Elemente Hilfslinse 28, Strahlenvertauscher 11 und Funduslinse 23 nicht vor das Hauptobjektiv 4 geschwenkt und somit kann auf herkömmliche Weise das Patientenauge 1 mit der Mikroskopbeleuchtung 8 durch die Pupille beleuchtet werden. Das heißt, dass nur der Lichtleiter 9 gespeist werden muss. Sofern in diesem Anwendungsmodus eine gleichzeitige oder ausschließliche transsklerale Beleuchtung via Lichtleiter 16 von Vorteil ist, sollen die beiden Beleuchtungsarten erfindungsgemäß frei kombinierbar sein.

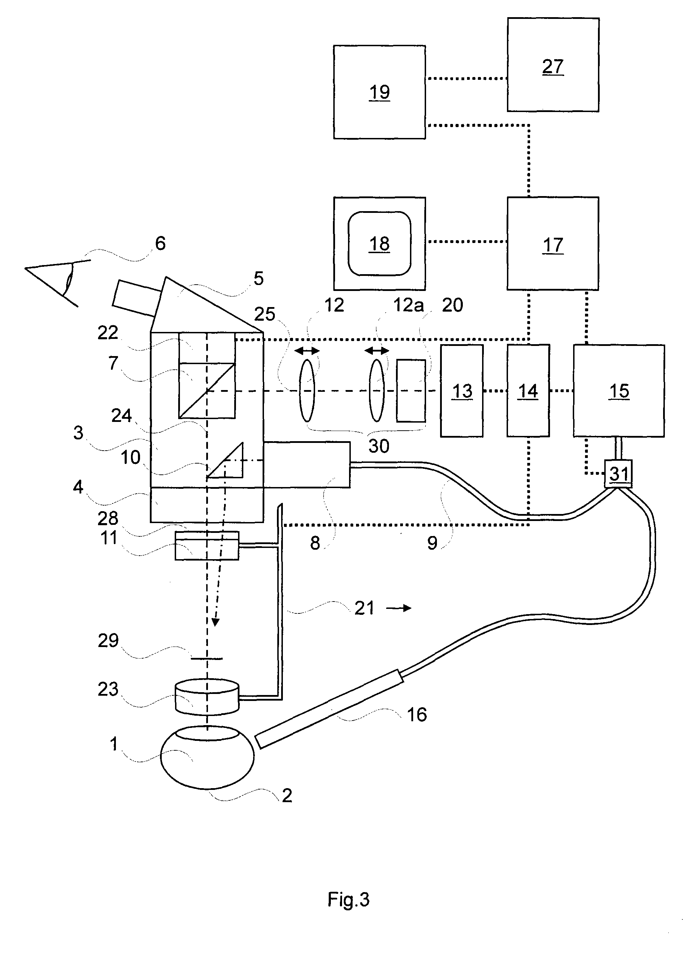

In Fig.3 ist symbolisch dargestellt, wie mittels einer durch den Rechner 17 gesteuerten optischen Lichtweiche 31 die erforderliche Beleuchtung geschaltet werden kann.

### Bezugszeichenliste

- 1 -: Patientenauge
- 2 -: Fundus
- 3 -: Operationsmikroskop
- 4 -: Hauptobjektiv
- 5 -: Stereo-Tubus
- 6 -: Beobachterauge
- 7 -: Strahlenteiler
- 8 -: Mikroskopbeleuchtung
- 9 -: Lichtleiter
- 10 -: Umlenkelement
- 11 -: Strahlenvertauscher
- 12, 12a -: Linse bzw. Linsengruppe
- 13 -: Kamera
- 14 -: Bildaufbereitung
- 15 -: Lichtquelle
- 16 -: Lichtleiter
- 17 -: Rechner
- 18 -: Monitor
- 19 -: Datenausgabe
- 20 -: Umlenkelement
- 21 -: Mechanische Einschwenkvorrichtung
- 22 -: Strahlenvertauscher (alternativer Anbringungsort zu 11)
- 23 -: Funduslinse
- 24 -: Beobachtungsstrahlengang
- 25 -: Ausgekoppelter Kamera-Strahlengang
- 26 -: Mikroskoplichtquelle
- 27 -: Zentrale Dokumentenverwaltung
- 28 -: Hilfslinse
- 29 -: Zwischenbild
- 30 -: Abbildungssystem
- 31 -: Optische Lichtweiche

## Patentansprüche

1. Operationsmikroskop (3), das einen Beobachtungsstrahlengang (24) definiert, in dem ein Strahlenteiler (7) vorgesehen ist, wobei durch den Strahlenteiler (7) ein Kamera-Strahlengang (25) definiert ist und wobei im Kamera-Strahlengang (25) eine Kamera (13) angeordnet ist, **dadurch gekennzeichnet, dass** das Operationsmikroskop (3) an einer Schnittstelle zwischen einer digitalen Funduskamera (13) und einer Funduslinse (23) eines modifizierten Netzhaut-Diagnosegeräts angeordnet ist.

2. Operationsmikroskop (3), das einen Beobachtungsstrahlengang (24) definiert, in dem ein Strahlenteiler (7) vorgesehen ist, wobei durch den Strahlenteiler (7) ein Kamera-Strahlengang (25) definiert ist und wobei im Kamera-Strahlengang (25) eine Kamera (13) angeordnet ist, **dadurch gekennzeichnet, dass** die Kamera (13) eine digitale Funduskamera (13) eines modifizierten Netzhaut-Diagnosegeräts ist.

3. Operationsmikroskop nach Anspruch 1 oder 2, **dadurch gekennzeichnet, dass** wenigstens eine Schaltvorrichtung vorgesehen ist, mittels der ein Anwender wahlweise zwischen einem Operationsmikroskop-Modus oder einem Netzhaut-Diagnosegerät-Modus wählen kann.

4. Operationsmikroskop (3) nach einem der vorhergehenden Ansprüche, **dadurch gekennzeichnet, dass** ein Lichtleiter (16) für eine transsklerale Netzhautbeleuchtung vorgesehen ist.

5. Operationsmikroskop (3) nach einem der vorhergehenden Ansprüche, **dadurch gekennzeichnet, dass** mindestens eine Beleuchtungsquelle (15) mit mindestens zwei Farbdioden versehen ist.

6. Operationsmikroskop nach Anspruch 4, **dadurch gekennzeichnet, dass** die Farbdioden gepulst ansteuerbar sind.

7. Operationsmikroskop (3) nach einem der vorhergehenden Ansprüche, **dadurch gekennzeichnet, dass** in dem Kamera-Strahlengang (25) - vorzugsweise zwischen dem Strahlenteiler (7) und der Kamera (13) - ein Abbildungssystem (30) mit verstellbaren Linsen oder -gruppen (12, 12a) angeordnet ist.

8. Operationsmikroskop (3) nach einem der vorhergehenden Ansprüche, **dadurch gekennzeichnet, dass** das Abbildungssystem (30) nur für die Wellenlängen der mindestens zwei Farbdioden der Beleuchtungsquelle (15) berechnet und korrigiert ist.

9. Operationsmikroskop (3) nach einem der vorhergehenden Ansprüche, **dadurch gekennzeichnet, dass** für die Korrektur der Bildqualität des Kamera-Strahlengangs (25) mit Hilfe des Abbildungssystems (30) vorzugsweise eine Kombination mit diffraktiven Elementen und klassischen Linsen vorgesehen ist.

10. Operationsmikroskop (3) nach einem der vorhergehenden Ansprüche, **dadurch gekennzeichnet, dass** die Kamera eine CCD-Kamera mit einer Vielzahl von Pixeln ist, **und dass** der das Abbildungssystem (30) verlassende Strahlverlauf nahezu senkrecht auf die Oberfläche der Pixel des oder der CCDs ausgerichtet ist.

11. Operationsmikroskop (3) nach einem der vorhergehenden Ansprüche, **dadurch gekennzeichnet, dass** eine Funduslinse (23) zwischen einem Hauptobjektiv (4) und einem Patientenauge (1) einschwenkbar ist.

12. Operationsmikroskop (3) nach einem der vorhergehenden Ansprüche, **dadurch gekennzeichnet, dass** das Operationsmikroskop (3) ein Stereo-Operationsmikroskop ist.

13. Operationsmikroskop (3) nach einem der vorhergehenden Ansprüche, **dadurch gekennzeichnet, dass** ein Strahlenvertauscher (11) und/oder eine Hilfslinse (28) mit einer mechanischen Einschwenkvorrichtung (21) zwischen dem Hauptobjektiv (4) und dem Patientenauge (1) einschwenkbar sind.

14. Operationsmikroskop (3) mit einer Mikroskopbeleuchtung (8) nach einem der vorhergehenden Ansprüche, **dadurch gekennzeichnet, dass** die Mikroskopbeleuchtung (8) beim Betrieb der Kamera (13) ausschaltbar ist.

15. Operationsmikroskop (3) nach einem der vorhergehenden Ansprüche, **dadurch gekennzeichnet, dass** die Beleuchtungsquelle (15) durch einen Rechner (17) gesteuert bei Aufnahmen der Kamera (13) automatisch zuschaltbar ist.

16. Operationsmikroskop (3) nach einem der vorhergehenden Ansprüche, **dadurch gekennzeichnet, dass** die Pulsfrequenz der Beleuchtungsquelle (15) oberhalb der Flimmergrenze des Beobachterauges (6) liegt.

17. Operationsmikroskop (3) nach einem der vorhergehenden Ansprüche, **dadurch gekennzeichnet, dass** die Beleuchtungsquelle (15) sowohl als Kamerabeleuchtung als auch als Beleuchtung für das Operationsmikroskop (3) einsetzbar ist und wahlweise ihr Licht einem Lichtleiter (9) für eine herkömmliche Mikroskopbeleuchtung (8) und/oder einem Lichtleiter (16) für eine transsklerale Netzhautbeleuchtung zuführbar ist.

18. Operationsmikroskop (3) nach einem der vorhergehenden Ansprüche, **dadurch gekennzeichnet, dass** eine optische Lichtweiche (31 ) zur Schaltung des Lichts aus der Beleuchtungsquelle (15) auf den Lichtleiter (9) für die herkömmliche Mikroskopbeleuchtung und/oder auf den Lichtleiter (16) für die transsklerale Netzhautbeleuchtung angeordnet ist.

19. Operationsmikroskop (3) nach einem der vorhergehenden Ansprüche, **dadurch gekennzeichnet, dass** ein Rechner (17) vorgesehen ist durch den das Licht auf den Lichtleiter (9) für die herkömmliche Mikroskopbeleuchtung und/oder den Lichtleiter (16) für die transsklerale Netzhautbeleuchtung schaltbar ist.

20. Operationsmikroskop (3) nach einem der vorhergehenden Ansprüche, **dadurch gekennzeichnet, dass** die Funduslinse (23), der Strahlenvertauscher (11 ) und die Hilfslinse (28), durch den Rechner (17) gesteuert, automatisch zuschaltbar bzw. einschwenkbar sind.

21. Operationsmikroskop (3) nach einem der vorhergehenden Ansprüche, **dadurch gekennzeichnet, dass** der Strahlenvertauscher (11) im Stereo-Operationsmikroskop (3) an einem Anbringungsort (22) zwischen einem Stereo-Tubus (5) und dem Strahlenteiler (7) angeordnet ist **und** vorzugsweise durch den Rechner (17) gesteuert und automatisch zuschaltbar ist.

22. Operationsmikroskop (3) nach einem der vorhergehenden Ansprüche, **dadurch gekennzeichnet, dass** die Funduslinse (23) für direkten Kontakt mit dem Patientenauge (1) ausgebildet ist.

23. Operationsmikroskop (3) nach einem der vorhergehenden Ansprüche, **dadurch gekennzeichnet, dass** die Funduslinse (23) im Betrieb für einen Abstand zu dem Patientenauge (1) ausgebildet ist.
